# EUROPÄISCHE PATENTSCHRIFT

(11) **EP 0 829 500 B1**
(45) Veröffentlichungstag und Bekanntmachung des Hinweises auf die Patenterteilung: **03.03.2004**
(21) Anmeldenummer: 97115092.5
(22) Anmeldetag: 01.09.1997
(51) Int. Cl.: C08G 18/80, C08K 5/3435

(54) **Stabilisierte blockierte Isocyanate**
Stabilised blocked isocyanates
Isocyanates bloqués stabilisés

(30) Priorität: 13.09.1996 DE 19637334
(43) Veröffentlichungstag der Anmeldung: 18.03.1998
(73) Patentinhaber: Bayer MaterialScience AG, 51368 Leverkusen (DE)
(72) Erfinder: König, Eberhard, Dr., 51375 Leverkusen (DE); Noble, Karl-Ludwig, Dr., 51467 Bergisch Gladbach (DE); Engbert, Theodor, Dr., 50968 Köln (DE)

(56) Entgegenhaltungen:
- EP-A- 0 475 003
- EP-A- 0 654 490
- US-A- 5 142 001

## Beschreibung

Die Erfindung betrifft neue, gegenüber Thermovergilbung stabilisierte blockierte Polyisocyanate, deren Herstellung und Verwendung.

Blockierte Polyisocyanate werden in 1K-PUR-Einbrennlacken, insbesondere bei Automobil-Klarlacken und für Coil Coating-Lacke eingesetzt. Für diesen Einsatzzweck ist nur eine geringfügige Thermovergilbung der Lacke, insbesondere unter Übertrennbedingungen zugelassen.

Einen mehr oder weniger großen Beitrag zur Thermovergilbung von blockierten Isocyanaten liefert dabei das Blockierungsmittel. Blockierungsmittel, die nur eine sehr geringe Thermovergilbung verursachen, sind bekannt. Es handelt sich hierbei um Dimethylpyrazol und 1,2,4-Triazol. Beide Blockierungsmittel haben aber spezifische Nachteile. Das erstgenannte ist relativ teuer, das zweite ist nicht allgemein einsetzbar. Mit den technisch wichtigsten Lackpolyisocyanaten auf Basis von HDI führt 1,2,4-Triazol zu stark kristallisierenden, also für Lackanwendungen ungeeigneten Produkten.

Demgegenüber wäre Butanonoxim ein wirtschaftlich und lacktechnisch gut einsetzbares Blockierungsmittel, wenn man die von ihm verursachte Thermovergilbung deutlich mindern könnte.

Ein Stabilisierungsmittel, dass die Thermovergilbung auch von Butanonoxim blockierten Isocyanaten deutlich herabsetzt, ist bekannt (z.B. US-A 5 216 078 und der EP-A 654 490 und handelsüblich, Elf-Atochem, ®Luchem HA-R 100):

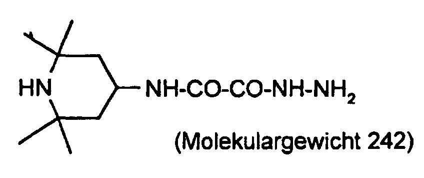

EP-A 0 475 003 lehrt die Verwendung von sterisch gehinderten Piperidin-Derivaten als Blockierungsmittel für Polyisocyanate, die sich bereits bei Raumtemperatur wieder deblockieren lassen.

Aufgabe der Erfindung war es daher, nach alternativen Wegen zu suchen, um Butanonoxim blockierte Isocyanate in wirtschaftlich und technisch gut durchführbarer Weise gegen Thermovergilbung zu schützen.

Gegenstand der Erfindung sind (cyclo)aliphatische Polyisocyanate, deren Isocyanatgruppen zu mindestens 95 % in mit Blockierungsmitteln blockierter Form vorliegen und die einen Gehalt an unblockierten und blockierten Isocyanatgruppen (berechnet als NCO) von insgesamt 5 - 25 Gew.-% aufweisen, dadurch gekennzeichnet, dass die blockierten Polyisocyanate einen Gehalt, bezogen auf Feststoff, an den folgenden stabilisierenden Verbindungen aufweisen:
A) 0,1 - 5,0 Gew.-% des "HALS-Amins" der Formel (I) 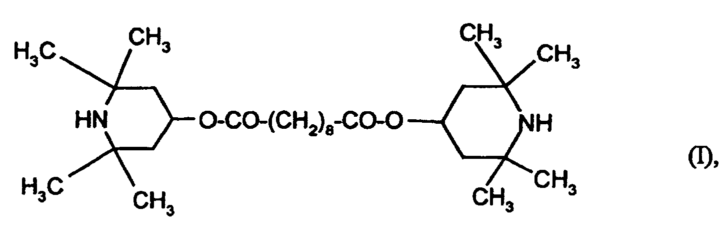 welches keine Hydrazidgruppen aufweist und gegebenenfalls
B) 0,1 - 8,0 Gew.-% an Hydraziden mit der Struktureinheit der Formel (II) 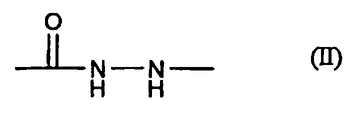 und
C) 0 - 5,0 Gew.-% an anderen, von A) und B) verschiedenen Stabilisatoren.

Gegenstand der Erfindung ist auch ein Verfahren zur Herstellung dieser blockierten Polyisocyanate, gegebenenfalls in Lacklösemitteln gelöster Form, durch Umsetzung einer, gegebenenfalls in Lacklösemitteln gelösten Polyisocyanat-Komponente mit Blockierungsmitteln, dadurch gekennzeichnet, dass vor, während oder im Anschluss an die Blockierungsreaktion
A) 0,1 - 5,0 Gew.-% des "HALS"-Amins der Formel (I)
   und gegebenenfalls
B) 0,1 - 5,0 Gew.-% Hydrazide mit der Struktureinheit der Formel (II) 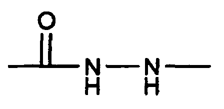 und
C) 0 - 5,0 Gew.-% andere, von A) und B) verschiedene Stabilisatoren
hinzugemischt bzw. eingebaut/abreagiert werden.

Gegenstand der Erfindung ist schließlich auch die Verwendung der erfindungsgemäßen blockierten Polyisocyanate als Vernetzer für organische Polyhydroxylverbindungen in Polyurethan-1K-Einbrenulacken, beispielsweise für Automobil-Klarlacke oder Coil Coating.

Die mit den erfindungsgemäß stabilisierten Polyisocyanaten erreichten Vorteile bestehen, wie bereits zum Teil angedeutet, in einer deutlichen Verbesserung der Vergilbungsresistenz beim
- Überbrennen (z.B. 30' 160°C oder Peak Metal Temperature >254°C)
- Wärmetempern (z.B. 120 h bei 120°C, vgl. Beispiel 3).

Mit anderen Worten kann bei Einsatz der erfindungsgemäßen Stabilisierung einfach zugängliche Blockierungsmittel, wie z.B. Butanonoxim oder Diisopropylamin, verwenden und vergleichbar geringe Thermovergilbungswerte erzielen, wie sie sonst nur mit aufwendig herstellbaren Blockierungsmitteln, z.B. Dimethylpyrazol, erreichbar sind.

Bei den erfindungsgemäßen blockierten Polyisocyanaten zugrundeliegenden Polyisocyanaten handelt es sich um an sich bekannte Lackpolyisocyanate mit aliphatisch und/oder cycloaliphatisch gebundenen Isocyanatgruppen und einem Isocyanatgehalt von 7 bis 30, vorzugsweise 12 bis 25 Gew.-%. In Betracht kommen insbesondere die an sich bekannten, Biuret-, Isocyanurat- und/oder Uretdiongruppen aufweisenden Lackpolyisocyanate auf Basis von 1,6-Diisocyanatohexan (HDI), 1-Isocyanato-3,3,5-trimethyl-5-isocyanatomethyl-cyclohexan (IPDI und/oder 4,4'-Diisocyanatodicyclohexylmethan. Besonders bevorzugt sind Isocyanuratgruppen aufweisende Lackpolyisocyanate auf Basis von (i) IPDI, (ii) 4,4'-Diisocyanatodicyclohexylmethan und (üi) 1,6-Diisocyanatohexan oder beliebige Gemische dieser Polyisocyanate.

Als Blockierungsmittel können beispielsweise Butanonoxim, Diisopropylamin, 1,2,4-Triazol, Imidazol, Malonester, Acetessigester, Dimethylpyrzol, ε-Caprolactam oder beliebige Gemische dieser Blockierungsmittel eingesetzt werden. Besonders bevorzugt ist Butanonoxim.

Das Stabilisierungsmittel A) der Formel (I) ist ein sogenanntes HALS (hindered amine light stabilizer)-Amin. Es wird u.a. von der Fa. Novartis unter der Bezeichnung Tinuvin® 770 DF vertrieben,

Als Stabilisierungsmittel B) der Formel (II) können Säurehydrazide, z.B. Essigsäurehydrazid oder Adipinsäuredihydrazid, wie sie in der US-A 5 216 078 genannt werden, oder auch Hydrazin-Addukte aus Hydrazin und cyclischen Carbonaten, wie sie in der EP-A 654 490 genannt werden, eingesetzt werden.

Besonders bevorzugt ist das Addukt aus 2 Mol Propylencarbonat und 1 Mol Hydrazin der Formel (IV) nachfolgender Struktur:

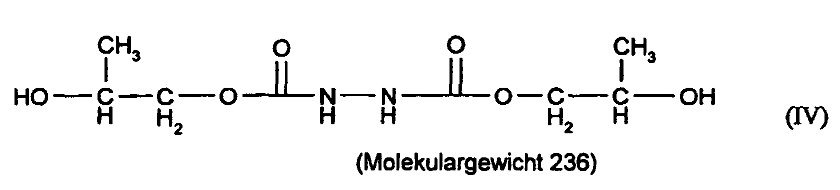

Als von A) und B) verschiedene Stabilisierungsmittel C), die gegebenenfalls mitverwendet werden können, zählen Antioxidantien wie 2,6-Di-tert-butyl-4-methyl-phenol, UV-Absorber vom Typ der 2-Hydroxyphenylbenzotriazole oder Lichtschutzmittel vom Typ der am Stickstoff substituierten HALS-Verbindung wie Tinuvin® 292.

Die Durchführung des erfindungsgemäßen Verfahrens erfolgt im Temperaturbereich von 20 bis 120°C, vorzugsweise 70 bis 90°C, in Substanz oder in geeigneten Lösemitteln wie beispielsweise n-Butylacetat, Methoxypropylacetat, Toluol oder höheren aromatischen Lösemittelgemischen wie sie beispielsweise von der Firma Exxon-Chemie unter der Bezeichnung Solvesso® vertrieben werden.

Beispielsweise kann das erfindungsgemäße Verfahren nach folgender Verfahrensvariante durchgeführt werden: Man legt 1,0 NCO-Äquivalent der Isocyanatkomponente gelöst vor und gibt hierzu eine Teilmenge des Blockierungsmittels von z.B. 0,8 Äquivalenten und lässt bei ca. 70°C abreagieren. Danach fügt man 1,0 - 1,5 %, bezogen auf den blockierten Vemetzer ohne Lösemittel, der Stabilisierungskomponente A) und beispielsweise 0,1 - 0,15 OH-Äquivalente der oben genannten Hydrazid-Stabilisierungskomponente B), gebildet aus 1 Mol Hydrazin und 2 Mol Propylencarbonat, hinzu und setzt mehrere Stunden bei ca. 85°C um, bis der kalkulierte NCO-Gehalt erreicht ist. Danach wird der verbleibende Anteil von NCO-Gruppen, 0,1 bis 0,05 Äquivalente, mit der entsprechenden Menge an weiterem Blockierungsmittel umgesetzt, bis kein NCO-Gehalt IR-Spektroskopisch mehr nachweisbar ist. Gegebenenfalls mit Lösemittel wird zum Schluss auf die gewünschte Viskosität eingestellt.

Die erfindungsgemäßen, überwiegend bzw. vollständig blockierten Polyisocyanate stellen wertvolle Vernetzerharze für organische Polyhydroxylverbindungen bei der Herstellung von Einbrennlacken dar. Sie können hierbei anstelle der bislang für diesen Zweck eingesetzten blockierten Polyisocyanate eingesetzt werden. Geeignete Polyhydroxylverbindungen für diesen Einsatzzweck, sowie weitere Details bezüglich der Herstellung und Anwendung derartiger Einbrennlacke können der einschlägigen Literatur entnommen werden. Besonders bevorzugtes Anwendungsgebiet für die erfindungsgemäßen Produkte ist ihre Verwendung als Vernetzter für Einbrenn-Polyurethanklarlacke, wie sie als Decklack zum einen bei der Automobil-Mehrschichtlackierung und zum anderen bei Coil Coating-Beschichtungen für "weiße Ware" zur Anwendung gelangen, wobei als Reaktionspartner für die erfindungsgemäßen blockierten Polyisocyanate die an sich bekannten Polyesterpolyole, Polyacrylatpolyole oder deren Gemische verwendet werden.

### Beispiele

In den nachfolgenden Beispielen beziehen sich alle Prozentangaben, soweit nicht anderslautendes angemerkt, auf das Gewicht.

### Beispiele

### Beispiel 1 (erfindungsgemäß)

Herstellung eines erfindungsgemäßen, stabilisierten Polyisocyanats, enthalten sind 1,5 % einer am N-Atom nicht substitutierten HALS-Verbindung und 4,4 % einer Hydrazid-Verbindung.

### Ansatz:

| | |
|---|---|
| 400,0 g (2,0 Val) | eines isocyananurathaltigen Lackpolyisocyanates auf Basis von HDI, NCO-Gehalt 21 % |
| 154,9 g (1,78 Val) | Butanonoxim |
| 26,0 g (0,22 Val) | des oben genannten Hydrazin-Adduktes aus 1 Mol Hydrazin-hydrat und 2 Mol Propylencarbonat vom Molekulargewicht 236 |
| 9,0 g | Tinuvin® 770 DF |
| 168,4 g | Methoxypropylacetat |
| 84,4 g | Solvesso® 100 |
| 842,7 g (1,78 Val) | blockierter NCO-Vernetzter blockierter NCO-Gehalt Ber.: 8,8 % |

### Durchführung:

Das obige Polyisocyanat wird in Methoxypropylacetat vorgelegt und auf ca. 60°C vorgewärmt. Die Apparatur wird mit Stickstoff gespült. Zu der gerührten Lösung wird in Portionen eine Teilmenge von 146 g (1,68 Val) Butanonoxim gegeben. Man setzt ca. 1 h bei 80°C um, bis ein NCO-Gehalt von ca. 1,9 % (Ber. 1,88 %) erreicht wird. Danach wird das zähflüssige Hydrazin-Addukt in einem Guss und die Gesamtmenge des pulverformigen Tinuvin® 770 DF eingerührt und die Innentemperatur auf 90°C gesteigert. Man setzt ca. 10 Stunden bei 90°C um, bis ein NCO-Gehalt von 0,6 % (Ber. 0,56 % erreicht ist. Anschließend wird die Restmenge von 8,9 g Butanonoxim hinzugefügt und nach ca. 30 Min. weiteren Rührens ist IR-spektroskopisch kein NCO-Gehalt mehr feststellbar.

Man verdünnt mit Solvesso® 100 auf eine 70 %ige Lösung und erhält eine farblose klare Lösung mit einer Viskosität (23°C) und 2 600 mPa·s. Diese blockierte Polyisocyanat-Lösung hat ein NCO-Äquivalentgewicht von 477.

### Beispiel 2 (Vergleich)

Als Vergleich dient ein handelsübliches, mit Butanonoxim blockiertes Polyisocyanat auf Basis des isocyanurathaltigen Lackpolyisocyanats gemäß Beispiel 1, 75 %ig gelöst in Solvesso® 100. Das NCO-Äquivalentgewicht der Lösung (bezogen auf die blockierten Isocyanatgruppen) liegt bei 378. Die Lösung weist eine Viskosität (23°C) von 3 200 mPa·s auf.

### Beispiel 3

Es wird die Herstellung von Coil Coating Weiß-Decklacken und deren vergleichende Ausprüfung beschrieben.

Als OH-Komponente dient der ölfreie Polyester Alkylnol® 1665 der Bayer AG, 65 %ig in Solventnaphtha gelöst, mit einem OH-Äquivalentgewicht von 1000. Die Bindemittelkombination aus den blockierten Polyisocyanaten der Beispiele 1 und 2 mit Alkynol® 1665 im Verhältnis der Äquivalentgewichte sieht wie folgt aus:

### Bindemittelkombinationen

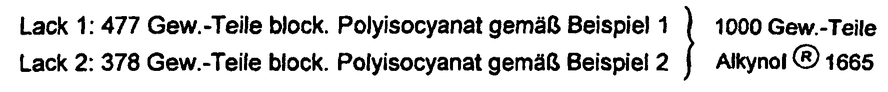

Als Pigmentanreibung wird folgender Stammansatz gemacht:

| | |
|---|---|
| 9,5 Gew.-Teile | Alkynol® 1665 |
| 8,1 Gew.-Teile | Solvesso® 200 S |
| 28,6 Gew.-Teile | Bayertitan® R-KB-4 |

werden mit 2 mm Siliquarzperlen ca. 1 Stunde lang auf dem Skandex Mischer dispergiert. Danach wird das Mahlgut mittels eines Siebes von den Glasperlen abgetrennt. Zu diesem Pigment-Stammansatz werden folgende übrige Lackkomponenten zugemischt:

| | |
|---|---|
| 21,1 Gew.-Teile | Alkynol® 1665 |
| für Lack 1 → 14,6 Gew.-Teile | blockiertes Polyisocyanat gemäß Beispiel 1 |
| für Lack 2 → 11,6 Gew.-Teile | blockiertes Polyisocyanat gemäß Beispiel 2 |
| 0,8 Gew.-Teile | DBTL, 10 %ig in Solvesso® 200 S |
| 7,0 Gew.-Teile | Celluloseacetobutyrat CAB 531-1 |
| 1,4 Gew.-Teile | Acrynal® 4 F 50 %ig in Solvesso® 200 S |

Mittels weiterer Mengen Solvesso® 200 S wird die Verarbeitungsviskosität auf ca. 70 sec. DIN 4/23°C eingestellt.

Die beiden Lacke 1 und 2 werden auf chromatierte Aluminiumbleche (1 mm dick) aufgeraktelt und sofort in Aalborgofen auf dem Drehteller eingebrannt.

| | | |
|---|---|---|
| PMT | 232°C | 40 sec. bei 350°C Ofentemperatur |
| PMT | >254°C | 50 sec. bei 350°C Ofentemperatur |

Die Filmtrockenschichtdicke beträgt 20 - 23 µm.

Folgende Eigenschaften wurden ermittelt:

| Eigenschaften | Lack 1 (erfindungsgemäß) | Lack 2 (Vergleich) |
|---|---|---|
| MEK-Doppelhübe | >100 | >100 |
| PMT/°C | 232 | 232 |
| Glanz 20/60° | 72/89 | 72/89 |
| Mikrohärte/µm | 3,1 | 5,0 |
| 10 g, 30 s/Rückstellung | 3,1 | 4,3 |
| Buchhotzhärte | 91 | 87 |
| Impact-Test/lb | 80 | 80 |
| Haftfestigkeit | 0 | 0 |
| T-bend-Test | T 1,0 | T 1,0 |
| Weißgrad | 91,0 | 91,2 |
| Gelbwert | -1,9 | -1,9 |
| Weißgrad PMT >254°C | 84,8 | 74,7 |
| Gelbwert PMT > 254°C | 0,6 | 4,2 |
| Weißgrad n. 120 h bei 120°C | 85,6 | 78,9 |
| Gelbwert n. 120 h bei 120°C | 0,2 | 2,8 |

Entscheidend sind die Eigenschaftsunterschiede am Ende der Tabelle, also unter Überbrennbedingungen bei einer "Peak Metal Temperature" >254°C. Bei dieser hohen Objekttemperatur entfaltet der Lack 1 mit dem erfindungsgemäßen Polyisocyanat seine größere Stabilität gegen Wärmevergilbung. Der Weißgrad, gemessen nach Berger, ist mit 84,8 Einheiten um ca. 10 Einheiten höher als bei Lack 2. Entsprechend geringer ist die Vergilbung (Gelbwert-Messing erfolgt nach DIN 6167) mit 0,6 gegenüber 4,2 bei Lack 2.

Ein Eigenschaftskriterium, das für "weiße Ware", z.B. Herde, wichtig ist, ist die 120°C-Temperung für 120 Stunden. Auch hierbei schneiden der Weiß- und Gelbwert des Lackes 1 mit dem erfindungsgemäßen Polyisocyanat deutlich besser ab.

## Patentansprüche

1. (Cyclo)aliphatische Polyisocyanate, deren Isocyanatgruppen zu mindestens 95 % in mit Blockierungsmitteln blockierter Form vorliegen und die einen Gehalt an unblockierten und blockierten Isocyanatgruppen (berechnet als NCO) von insgesamt 5 - 25 Gew.-% aufweisen, **dadurch gekennzeichnet, dass** die blockierten Polyisocyanate einen Gehalt, bezogen auf Feststoff, an den folgenden stabilisierenden Verbindungen aufweisen:
A) 0,1 - 5,0 Gew.-% Amin der Formel (I) 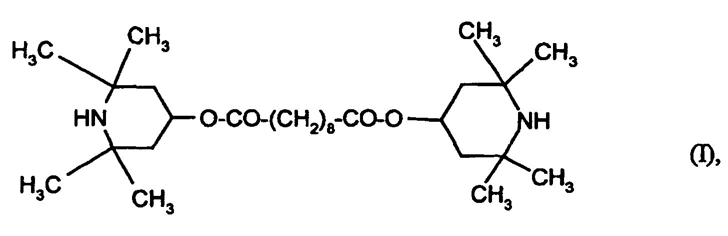 und gegebenenfalls
B) 0,1 - 8,0 Gew.-% an Hydraziden mit der Struktureinheit der Formel (II) 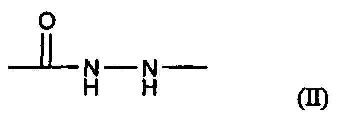 und
C) 0 - 5,0 Gew.-% an anderen, von A) und B) verschiedenen Stabilisatoren.

2. Verfahren zur Herstellung der Polyisocyanate nach Anspruch 1, gegebenenfalls in Lacklösemitteln gelöster Form, durch Umsetzung einer gegebenenfalls in Lacklösemitteln gelösten Polyisocyanat-Komponente mit Blockierungsmitteln, **dadurch gekennzeichnet, dass** vor, während oder im Anschluss an die Blockierungsreaktion
A) 0,1 - 5,0 Gew.-% des Amins der Formel (I) 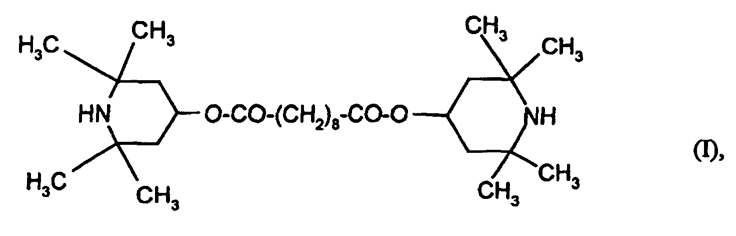 und gegebenenfalls
B) 0,1 - 8,0 Gew.-% Hydrazide mit der Struktureinheit der Formel (II) 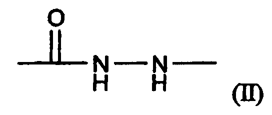 und
C) 0 5,0 Gew.-% andere, von A) und B)
verschiedene Stabilisatoren hinzugemischt bzw. eingebaut/abreagiert werden.

3. Verwendung der blockierten Polyisocyanate gemäß Anspruch 1 als Vemetzer für organische Polyhydroxylverbindungen in Polyurethan-1K-Einbrennlacken, beispielsweise für Automobil-Klarlacke oder Coil Coating.

## Claims

1. (Cyclo)aliphatic polyisocyanates whose isocyanate groups are present in at least 95 % in the form blocked with blocking agents and that contain an amount of unblocked and blocked isocyanate groups (calculated as NCO) of 5-25 wt.% in total, **characterised in that** the blocked polyisocyanates contain the following amounts, referred to solids, of the following stabilising compounds:
A) 0.1 - 5.0 wt.% of amines of the formula (I) 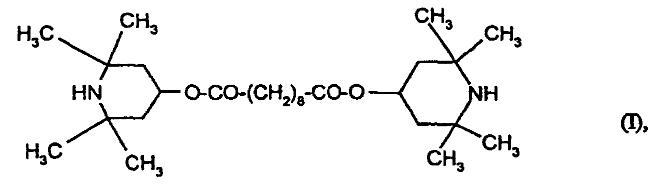 and optionally
B) 0.1 - 8.0 wt.% of hydrazides having the structural unit of the formula (II) 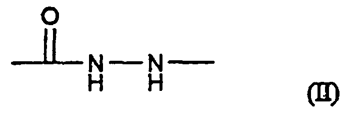 and
C) 0 - 5.0 wt.% of other stabilisers different
from A) and B).

2. Process for producing the polyisocyanates according to claim 1, optionally in a form dissolved in paint solvents, by reacting a polyisocyanate component, optionally dissolved in paint solvents, with blocking agents, **characterised in that** before, during or after the blocking reaction
A) 0.1 - 5.0 wt.% of the amine of the formula (I) 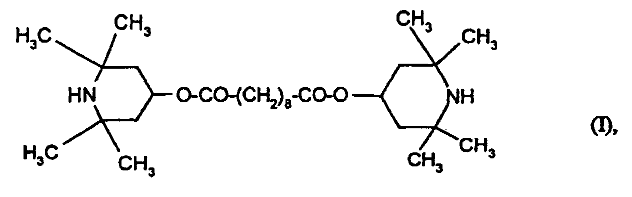 and optionally
B) 0.1 - 8.0 wt.% of hydrazides containing the structural units of the formula (II) 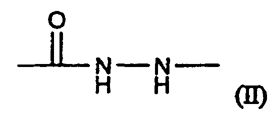 and
C) 0 - 5.0 wt.% of other stabilisers different from A) and B), are mixed in, added, incorporated or reacted therewith.

3. Use of the blocked polyisocyanates according to claim 1 as crosslinking agents for organic polyhydroxyl compounds in polyurethane 1C stoving lacquers, for example for automotive clear lacquers or coil coating.

## Revendications

1. Polyisocyanates (cyclo)aliphatiques dont les radicaux isocyanate sont présents à raison de 95% au moins sous forme bloquée à l'aide d'agents bloquants et qui présentent un taux de radicaux isocyanate bloqués et non bloqués (calculés comme du NCO) de 5 à 25 % en poids au total, **caractérisés en ce que** les polyisocyanates bloqués présentent un taux des composés stabilisateurs suivants, par rapport aux matières solides, de:
A) 0,1 à 5,0 % en poids de l'amine "HALS" de formule (I) 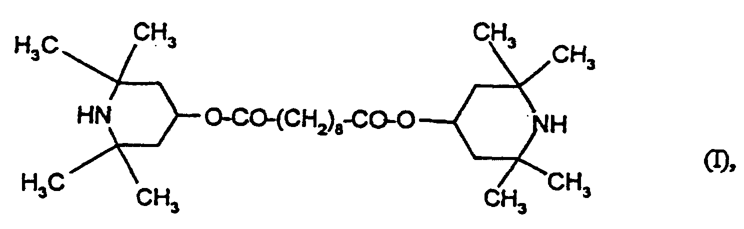 et, éventuellement,
8) 0,1 à 8,0% en poids d'hydrazides avec l'unité structurelle de formule (II) 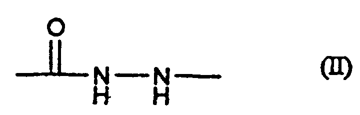 et
C) 0 - 5,0 % en poids d'autres stabilisateurs différant de A) et B).

2. Procédé de préparation des polyisocyanates bloqués selon le revendication 1, éventuellement sous forme dissoute dans des solvants pour vernis, par mise en réaction d'un composant polyisocyanate éventuellement dissous dans des solvants pour vernis avec des agents bloquants **caractérisé en ce que**, avant, pendant ou après la réaction de blocage,
A) 0,1 à 5,0 % en poids de l'amine "HALS" de formule (I) 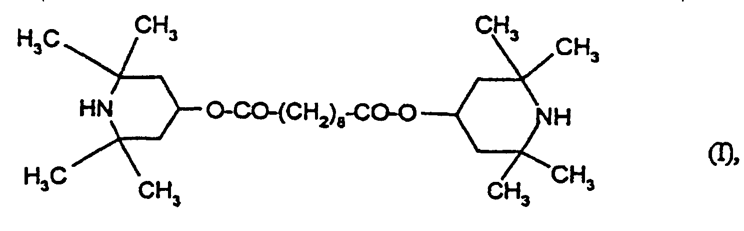 et éventuellement
B) 0,1 à 8,0% en poids d'hydrazides avec l'unité structurelle de formule (II) 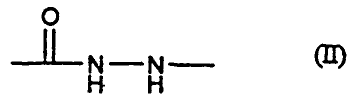 et
C) 0 - 5,0 % en poids d'autres stabilisateurs différant de A) et B)
sont ajoutés ou incorporés/amenés à réagir complètement.

3. Mise en oeuvre des polyisocyanates bloqués selon la revendication 1 comme des réticulants pour des composés polyhydroxylés organiques dans des vernis à cuire 1K de polyuréthane, par exemple pour des vernis transparents automobiles ou Coil Coating.
